# EUROPEAN PATENT SPECIFICATION

(11) **EP 0 287 328 B1**
(45) Date of publication and mention of the grant of the patent: **06.10.1993**
(21) Application number: 88303274.0
(22) Date of filing: 12.04.1988
(51) Int. Cl.: C07D 498/08, C07C 205/37, C07F 5/02

(54) **Preparation of chromogenic cryptahemispherands**
Herstellung von chromogenischen Cryptahemispheranden
Préparation de cryptahémisphérands chromogéniques

(30) Priority: 15.04.1987 US 38680
(43) Date of publication of application: 19.10.1988
(73) Proprietor: Bayer Corporation, Wilmington, MA 01887-1069 (US)
(72) Inventor: Czech, Bronislaw P., Peekskill, New York 10566 (US)
(74) Representative: Wain, Christopher Paul

(56) References cited:
- EP-A- 0 085 320
- EP-A- 0 223 613
- US-A- 4 156 683
- JOURNAL OF THE AMERICAN CHEMICAL SOCIETY, vol. 108, 1986; D.J. CRAM et al.: "Host-Guest complexation. 38. Cryptahemispherands and their complexes", pages 2989-2998
- THE JOURNAL OF THE AMERICAN CHEMICAL SOCIETY, vol. 64, 1942; P. BLOCK et al.: "The synthesis of 3',5'-Diiodothyronine", pages 1070-1074
- SYNTHETIC COMMUNICATIONS, vol. 11, no. 7, 1981; N. MIYAURA et al.: "The palladium-catalyzed cross-coupling reaction of phenylboronic acid with haloarenes in the presence of bases", pages 513-519

## Description

The present invention relates to a process for the preparation of certain chromogenic cryptahemispherands useful in the measurement of ions.

Certain terminology used herein has the following meaning:
The term "ionophore" includes, broadly, molecules capable of forming a complex with an ion in solution. For example the cyclic polypeptide, valinomycin, binds selectively to potassium ions in solution to form a cationic complex. Also included in this term are podands, corands, cryptands, hemispherands, cryptahemispherands, and spherands.

A "podand" is an organic linear compound containing donor or receptor atoms which has the capacity of associating with positively charged ions to form complexes.

The term "corands" refers to monocyclic compounds which contain electron donor atoms or acceptor atoms, which are electron rich or deficient, and which are capable of complexing with particular cations or anions because of their unique structures. Because of the unique sizes and geometries of particular corands, they are adaptable to complexing with various ions. In so complexing, the electron rich atoms, such as oxygens in a corand, become spatially oriented towards the electron deficient cation. The carbon atom segments of the cycle are simultaneously projected in a direction outwards from the ion. Thus, the resultant complex is hydrophilic in the center but is relatively hydrophobic at its perimeter.

"Cryptands" refers to polycyclic analogs of the corands. Accordingly, they include bicyclic and tricyclic multidentate compounds. In the cryptands, the cyclic arrangement of donor atoms is three dimensional in space, as opposed to the substantially planar configuration of the corands. A cryptand is capable of virtually enveloping the ion in three dimensional fashion and, hence, is capable of strong bonds to the ion in forming the complex. As with the corands, the donor atoms can include such atoms as oxygen, nitrogen and sulfur.

The term "hemispherands" refers to macrocyclic or macropolycyclic ionophore systems, whose cavities are partially preorganized for binding by the rigidity of the hydrocarbon support structure and the spatial and orientational dictates of appended groups.

The designation "cryptahemispherand" was given by Donald J. Cram in 1986 (Cram , et al., J. Am. Chem. Soc., 108 pp. 2998-3005 (1986) to the class of macrobicyclic compounds which show an extraordinary propensity for complexation of alkali metal cations. Cryptahemispherands combine the partially preorganized cavity features of the hemispherands, but contain multiple other ligand-gathering features of the cryptands. The generic structure of a cryptahemispherand is depicted, infra, as structure (I) 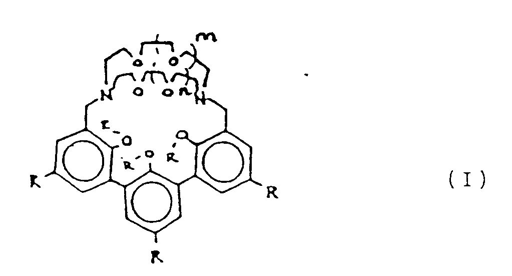
wherein:
R is hydrogen, alkyl, alkylidene, alkenyl, allyl, aryl or benzyl;
m is 0 to about 2; and
n is 0 to about 2;
Certain compounds were described in the literature prior to Cram, et al. supra, which are capable of not only behaving as ionophores by forming cation complexes but also, when complexed, exhibiting a detectable formation of, or change in, color.

Thus, experiments were published in 1977 whereby chromogenic moieties were covalently attached to ionophores to achieve a color change response to potassium (Takagi, et al., Analytical Letters, 10(3), pp. 1115-1122 (1977)). There it is taught to couple covalently a chromogenic moiety such as 4-picrylamino to an ionophore such as benzo-15-corand-5. Moreover, U.S. Patent No. 4,367,072 mentions many corands, cryptands and podands covalently substituted with a chromogenic group, such as 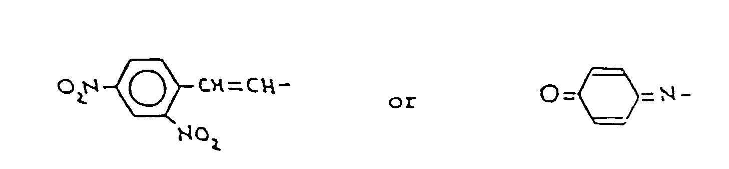
Yet another reference, German Offenlegungsschrift 3202779, published August 4, 1983 discloses a chromogenic cryptand structure.

Although the syntheses of non-chromogenic cryptahemispherands have been described by Cram et al., incorporation of a chromogenic moiety into the cryptahemispherand structure requires different synthetic strategy and has not been described before.

According to the present invention, there is provided a process for the preparation of a chromogenic cryptahemispherand (I) having the structure 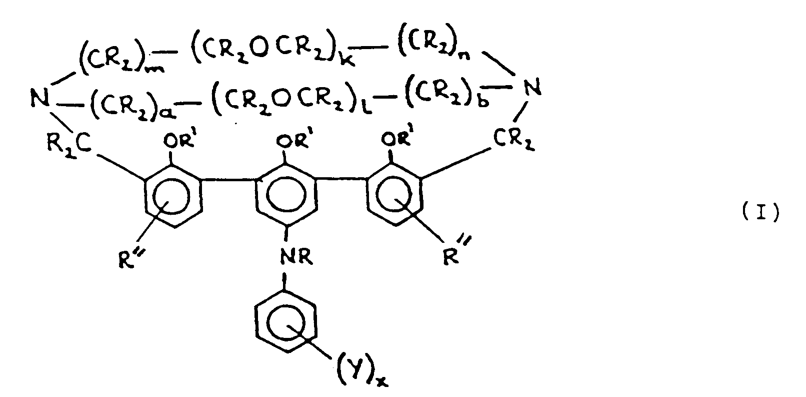
said process comprising the steps of:
(a) coupling a compound (II) having the structure 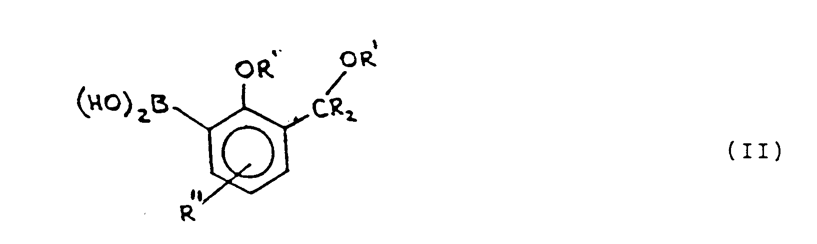 with a compound (III) having the structure 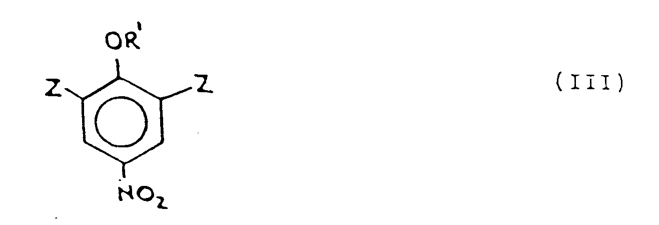 in the presence of a catalyst, to form a compound (IV) having the structure 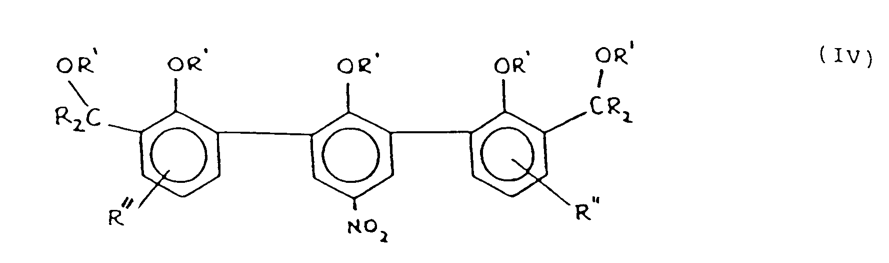
(b) subjecting the compound (IV) to reducing conditions to form a compound (V) having the structure 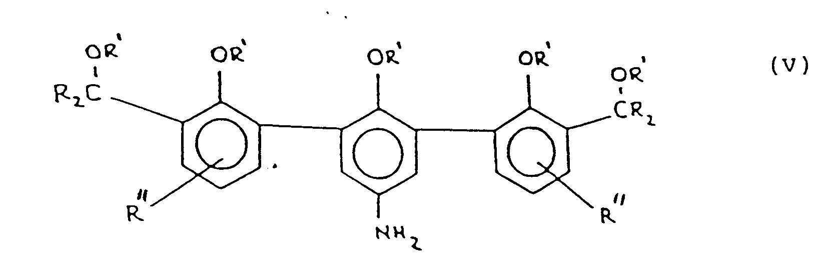
(c ) coupling the compound (V) to a compound having the structure 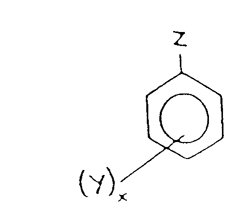 to form a compound (VI) having the structure 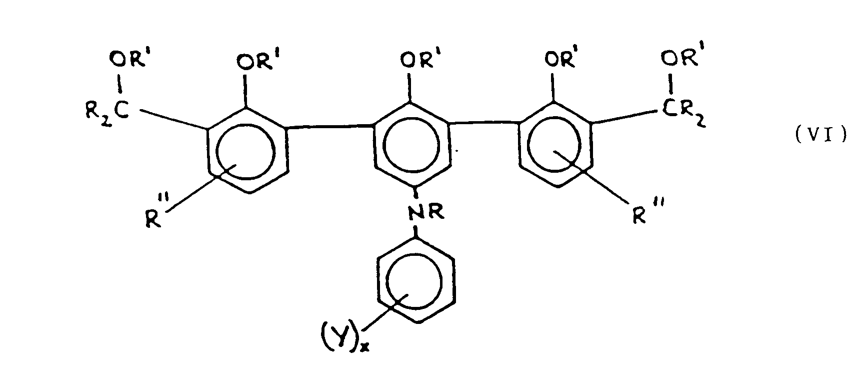
(d) halogenating the compound (VI) to form a compound (VII) having the structure 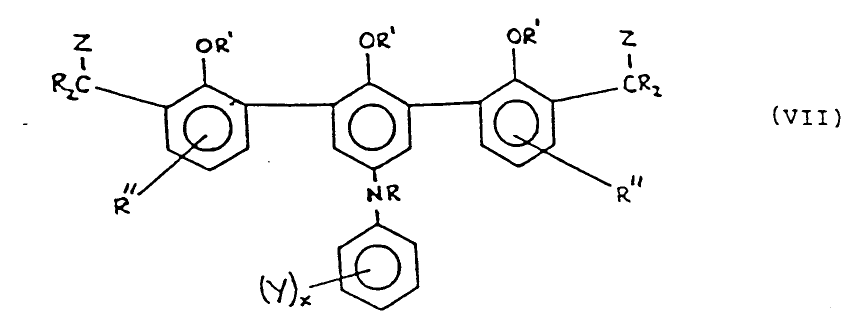
(e) forming the chromogenic cryptahemispherand (I) by coupling the compound (VII) with a diazacorand having the structure 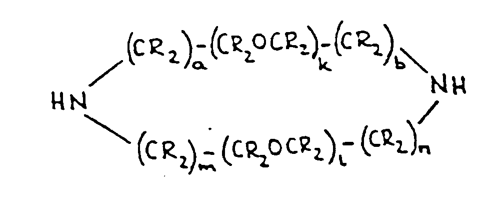 wherein each R is independently hydrogen, lower alkyl, lower alkylidene, lower alkenyl, allyl, aryl, or benzyl;
   each R' is independently lower alkyl, lower alkylidene, lower alkenyl, allyl, aryl, or benzyl;
   each R'' is independently hydrogen, lower alkyl, lower alkylidene, lower alkenyl, aryl or benzyl;
   each Y is independently an electron withdrawing group;
   each Z is halogen,
   a is 1 to 3;
   b is 1 to 3;
   k is 1 to 3;
   l is 1 to 3;
   m is 1 to 3;
   n is 1 to 3; and
   x is 2 to 4.

Examples of electron withdrawing groups are CN, NO₂, CF₃ and COOR. Reference may be made to Advanced Organic Chemistry, Reactions, Mechanisms, and Structure, (Third Edition) by Jerry March (John Wiley & Sons).

Preferably, compound II is prepared in a synthetic sequence comprising an alkylation or arylation reaction and a lithiation/boronation reaction in accordance with the reaction sequence: 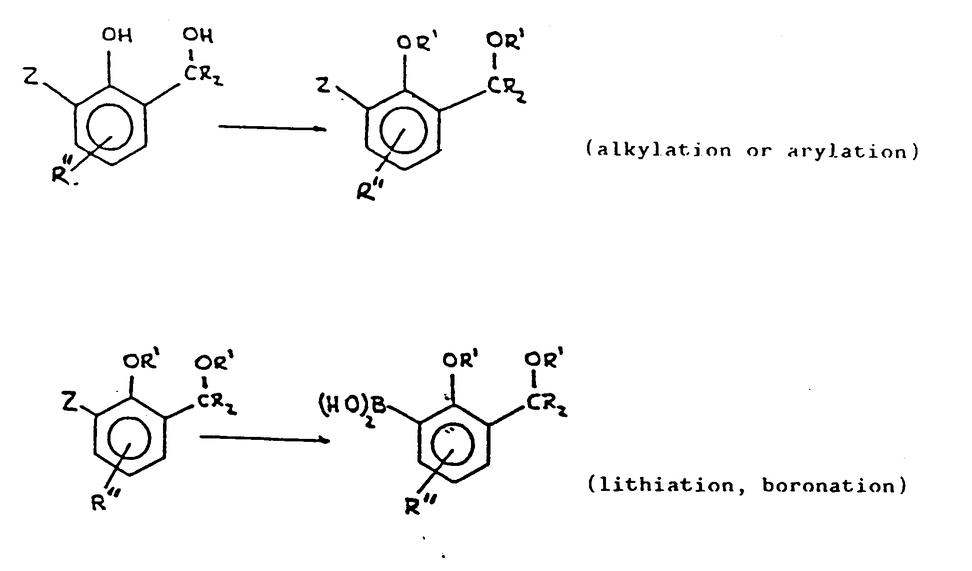
Preferably, compound III is prepared by an alkylation or arylation reaction in accordance with the reaction sequence:

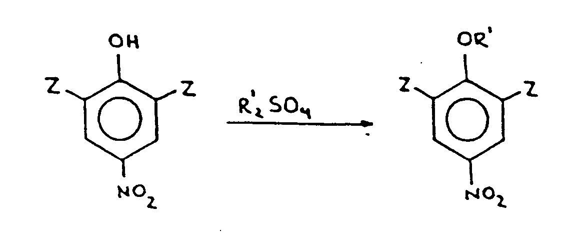

It is further preferred that the catalyst in coupling step (a) be tetrakis(triphenylphosphine)palladium.

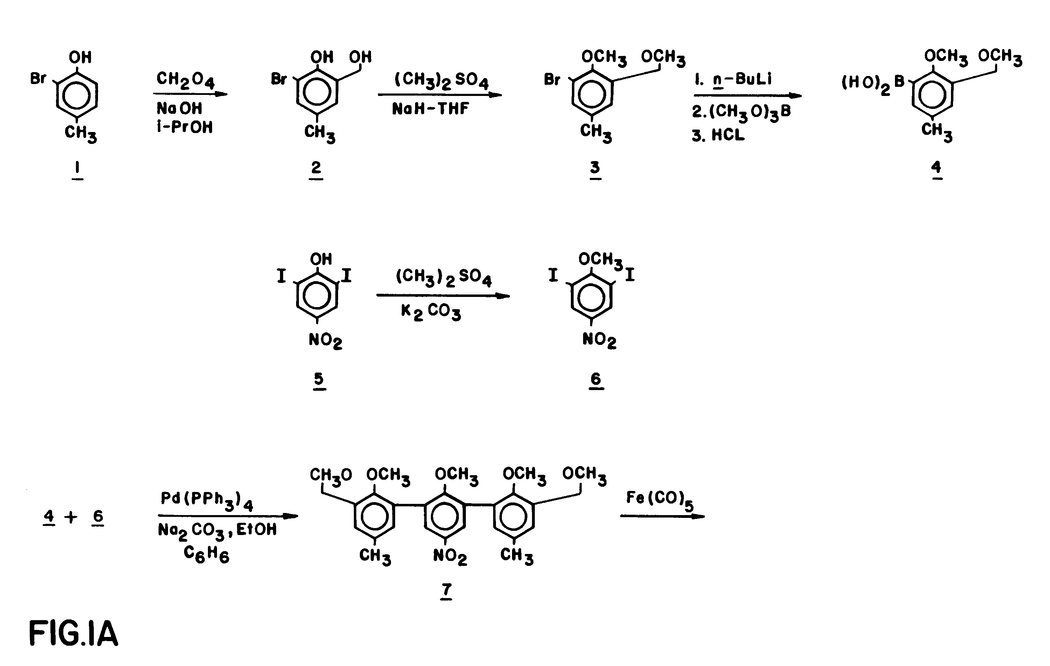

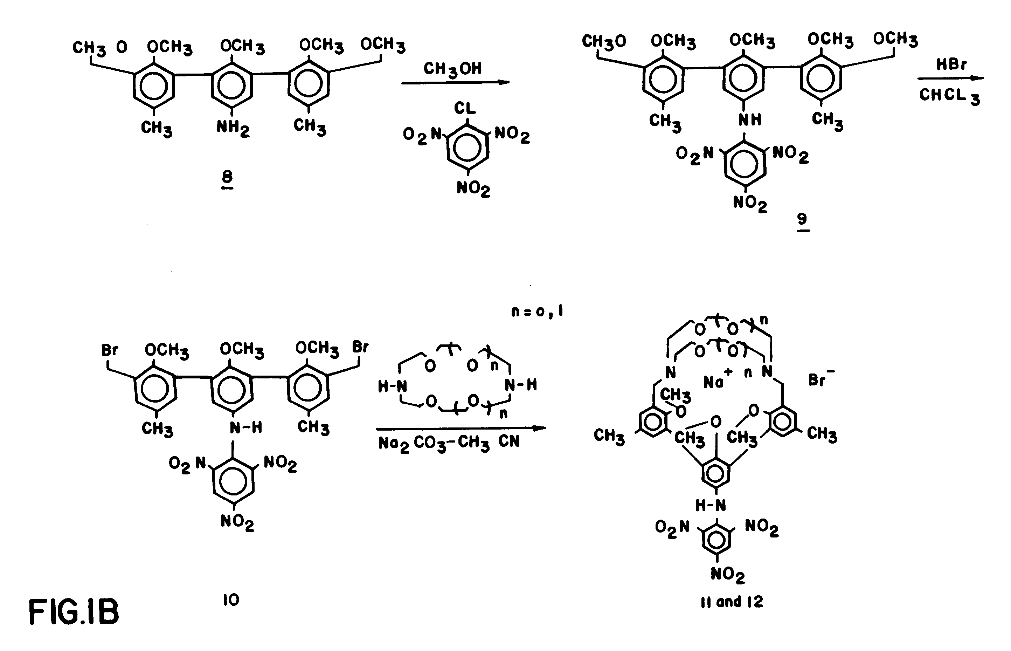

In the accompanying drawings, Figs. 1A and 1B illustrate one embodiment of the present invention. The invention will now be more particularly described with reference to the drawings.

### Preparation of Known Intermediate Compounds 2 and 6.

The 2-bromo-6-(hydroxymethyl)-4-methylphenol 2 used in the following preparation was obtained from commercially available 2-bromo-4-methylphenol 1 by the method described in the article by Cram, et. al., J. Am. Chem Soc., 106 pp. 4977-4987 (1984).

The 4-nitro-2,6-dimethylanisole 6 was prepared from commercially available 4-nitro-2,6-diiodophenol 5 by the method described in the article by Block, et. al., J. Am. Chem. Soc., 64 pp 1070-1074 (1942).

Reference should be made to these articles for further details.

### Preparation of Compound 3

To a solution of 2 (23.4g, 107.8 mmol) in 600 ml of THF under Ar at 0°C was added 15.2g (381 mmol) of 60% NaH. After warming to room temperature, 45.7g (360 mmol) of dimethyl sulfate was added and the mixture was refluxed 18 h, cooled to 0°C and methanol was added to decompose the excess NaH. The solvent was removed in vacuo to give a crude product which was dissolved in 100 ml of CHCl₃ and brine was added. The organic layer was separated, dried (MgSO₄) and evaporated. The residue was purified on a silica gel column (flash) with benzene-cyclohexane (1:4 ---> 1:1) to afford 23.7g (90%) of 3 as a colorless liquid.

The ¹H NMR spectrum (CDCl₃) gave absorptions at δ 2.29 (s, ArCH₃, 3H), 3.43 (s, OCH₃, 3H), 3.82 (s, OCH₃, 3H), 4.48 (s, ArCH₂, 2H), 7.14 (d, ArH, 1H), and 7.30 (d, ArH, 1H). Calcd. for C₁₀H₁₃BrO₂ (percent): C, 49.00; H, 5.35. Found (percent): C, 49.11; H, 5.34.

### Preparation of Compound 4

To a solution of 3 (13.0g, 53 mmol) in 200 ml of THF under Ar at -78°C was added 22.5 ml of 2.4 M n-BuLi (hexane). After stirring for 8 min, the lithiation solution was cannulated over 15 min into 48.0g (460 mmol) of trimethyl borate in 125 ml of THF at -78°C. The mixture was stirred 30 min. at -78°C over 45 min, diluted with 400 ml of 2N HCl, and stirred 1 h at 25°C. Ether (250 ml) was added, the mixture was stirred 6 h at 25°C, and the layers were separated. The aqueous layer was extracted with fresh ether (3x100 ml). The combined ether extracts were extracted with 3N aqueous NaOH (4x100 ml). The base extracts were cooled to 5°C and acidified to pH1 with concentrated HCl. Extraction of the aqueous solution with ether (3x100 ml) gave after evaporation of the solvent (room temperature, vacuum) 10.5g (95%) of a colorless viscous oil 4 which solidified during storage at -5°C and was used without further purification.

The ¹H NMR spectrum ((CD₃)₂CO) gave absorptions at δ 2.29 (s, ArCH₃, 3H), 3.38 (s, OCH₃, 3H), 3.80 (s, OCH₃, 3H), 4.45 (s, ArCH₂, 2H), 7.29 (d, ArH, 1H), and 7.52 (d, ArH, 1H).

### Preparation of Compound 7

To a mixture of 6 (4.00g, 9.9 mmol), and 4 (5.00g, 24.0 mmol) in 60 ml of toluene and 15 ml of ethanol was added under Ar 30 ml of 2M aqueous Na₂CO₃. To this vigorously stirred two-phase mixture was added 0.60g (0.52 mmol) of tetrakis(triphenylphosphine)palladium (0) and the mixture was refluxed for 45 h. The layers were separated and the organic layer was dried (MgSO₄), evaporated and the residue was column chromatographed on alumina with benzene, and benzene-ethyl acetate (20:1) to give 4.44g (93%) of 7 as a very viscous, pale yellow oil.

The mass spectrum (70eV) gave the expected molecular ion at m/e 481. The ¹H NMR spectrum (CDCl₃) gave absorptions at δ 2.36 (s, ArCH₃, 6H), 3.30 (s, OCH₃, 3H), 3.47 (s, OCH₃, 6H), 3.49 (s, OCH₃, 6H), 4.54 (s, ArCH₂, 4H), 7.12 (d, ArH, 2H), 7.28 (d, ArH, 2H), nd 8.25 (s, ArH, 2H).

| | | |
|---|---|---|
| Calcd. for C₂₇H₃₁NO₇ (percent): | C, 67.35, | H, 6.49. |
| Found (percent): | C, 67.27; | H, 6.38. |

### Preparation of Compound 8

To a mixture of 7 (4.65g, 9.7 mmol) in 175 ml of benzene and 175 ml of 1N NaOH under Ar was added 4.65g (23.7 mmol) of iron pentacarbonyl. The mixture was stirred for 18 h at room temperature, 500 ml of benzene was added, and the benzene layer was separated. The aqueous layer was extracted with benzene (2x100 ml), the combined organic layers were filtered through Celite (twice), dried (K₂CO₃), filtered and evaporated to a 70 ml volume and a residue was column chromatographed on silica gel (flash) with petroleum ether - ethyl acetate (3:1 ---> 1:1) to give 2.88g (66%) of 8 as a heavy, pale yellow oil which solidified during storage.

The mass spectrum (70 eV) gave the expected molecular ion at m/e 451). The ¹H NMR spectrum (CDCl₃) gave absorptions at δ 2.33 (s, ArCH₃, 6H), 3.14 (s, OCH₃, 3H), 3.45 (s, OCH₃, 6H), 3.51 (s, OCH₃, 6H), 4.54 (s, ArCH₂, 4H), 6.70 (s, ArH, 2H), 7.13 (s, ArH, 2H), and 7.19 (s, ArH, 2H).

Calcd. for C₂₇H₃₃NO₅ (percent): C, 71.82; H, 7.37 Found (percent): C, 71.75; H, 7.56.

### Preparation of Compound 9

A mixture of 8 (2.75g, 6.1 mmol), picryl chloride (2.00g, 8.1 mmol) and NaHCO₃ (0.51g, 6.1 mmol) in 325 ml of methanol under Ar at room temperature was stirred overnight, the solvent was removed in vacuo (room temp.), and a residue was dissolved in CHCl₃-H₂O (110 ml of each). The chloroform layer was dried (MgSO₄), concentrated to 10 ml and column chromatographed on silica gel (flash) with petroleum ether - ethyl acetate (2:1) to give 3.82g (95%) of 9 as a red foam.

The mass spectrum (70 eV) gave the expected molecular ion at m/e 662. The ¹H NMR spectrum (CDCl₃) gave absorptions at δ 2.35 (s, ArCH₃, 6H), 3.23 (s, OCH₃, 3H), 3.46 (s, OCH₃, 6H), 3.53 (s, OCH₃, 6H), 4.53 (s, ArCH₂, 4H), 7.09-7.25 (m, ArH, 6H), 9.08 (s, ArH, 2H), and 10.29 (s, NH, 1H).

Calcd. for C₃₃H₃₄N₄O₁₁ (percent): C, 59.81; H, 5.17. Found (percent): C, 59.86; H, 5.36.

### Preparation of Compound 10

Anhydrous HBr gas was bubbled into a solution of 9 (2.05g, 3.1 mmol) in 650 ml of CHCl₃ for 10 min. After stirring an additional 10 min the solution was poured into 800 ml of water and the mixture was stirred over 30 min. The organic layer was dried (MgSO₄), concentrated to 10 ml and column chromatographed on silica gel with CH₂Cl₂ to afford 1.79g (75%) of 10 as a red glass.

The ¹H NMR spectrum (CDCl₃) gave absorptions at δ 2.34 (s, ArCH₃, 6H), 3.23 (s, OCH₃, 3H), 3.62 (s, OCH₃, 6H), 4.61 (s, ArCH₂, 4H), 7.09-7.24 (m, ArH, 6H), 9.09 (s, ArH, 2H), and 10.30 (s, NH, 1H).

Calcd. for C₃₁H₂₈Br₂N₄O₉ (percent): C, 48.97; H, 3.71. Found: C, 48.70; H, 3.71.

### Cryptahemispherand 11

To a vigorously stirred solution containing 0.49 g (6.6 mmol) of anhydrous Li₂CO₃ in 100 ml of CH₃CN was added over a period of 20 h 1,7-dioxa-4,10-diazacyclododecane (0.22 g, 1.25 mmol) in CH₃CN (27 ml) and dibromide 10 (0.95 g, 1.25 mmol) in 27 ml of CH₃CN at reflux. After addition was completed reflux was continued for additional 15 h, then the solvent was removed in vacuo (25°C) and the residue was chromatographed on a silica gel column with CH₂Cl₂ - CH₃OH (95:5) to afford 0.54 g (56%) of an orange foam which is a complex of 11 with lithium bromide.

The ¹H NMR spectrum (CDCl₃) showed absorptions at δ 2.36 (s, ArCH₃, 6H), 2.53 (s, OCH₃, 3H), 2.36-2.72 (m, NCH₂, 8H), 3.12-4.19 (m, OCH₂, NCH₂, OCH₃, 18H), 7.05 (d, ArH, 2H), 7.14 (d, ArH, 2H), 7.28 (s, ArH, 2H) and 9.09 (s, ArH, 2H).

The cryptahemispherand 11 has the structure shown in Fig. 1 wherein n is 0.

### Cryptahemispherand 12

To a vigorously stirred solution containing 0.85g (8.0 mmol) of anhydrous Na₂CO₃ in 120 ml of CH₃CN was added over a period of 20h Kryptofix®22 (0.42g, 1.6 mmol) in CH₃CN (35 ml) and dibromide 10 (1.22 g, 1.6 mmol) in CH₃CN (35 ml) at reflux. After addition was completed reflux was continued for additional 15h, then the solvent was removed in vacuo (25°C) and the residue was chromatographed on a silica gel column with CH₂Cl₂ - CH₃OH (95:5 ---> 90:10) to give 1.30 g (84%) of 12 as a dark red powder. The product is a complex of 12 with NaBr.

The ¹H NMR spectrum (CDCl₃) showed absorptions at δ 2.36 (s, ArCH₃, 6H), 2.84 (s, OCH₃, 3H), 3.48 (s, OCH₃, 6H), 2.18-4.10 (m, NCH₂, OCH₂, 24H), 2.67 (d, ArCH₂N, 2H), 4.20 (d, ArCH₂N, 2H), 7.03 (d, ArH, 2H), 7.12 (d, ArH, 2H), 7.17 (s, ArH, 2H), and 9.09 (s, ArH, 2H).

The cryptahemispherand 12 has the structure shown in Fig. 1 wherein n is 1.

The compounds of formula I as defined herein are the subject of copending European patent application no. EP-A-0287329 filed on even date herewith.

The present invention has been particularly described with reference to the preparation of compounds 11 and 12. It is to be understood that all the other compounds falling within formula I as defined herein, can be made in essentially the same way by choosing the appropriate reactant(s) at each stage of the process.

## Claims

1. A process for the preparation of a chromogenic cryptahemispherand (I) having the structure 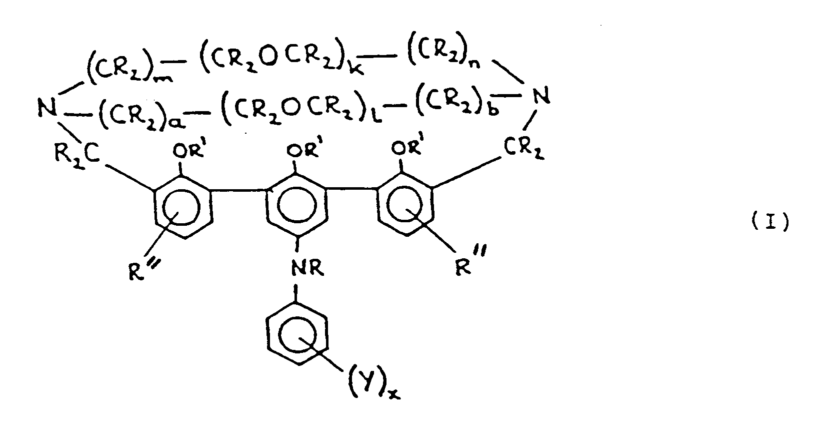 said process comprising the steps of:
(a) coupling a compound (II) having the structure 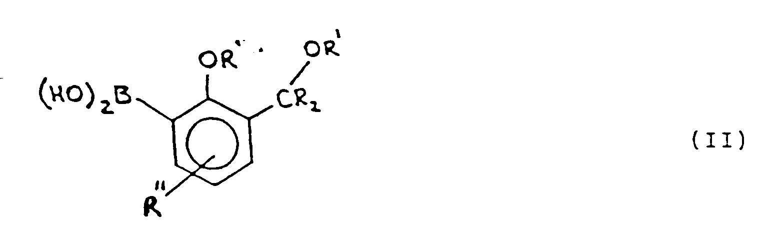 with a compound (III) having the structure 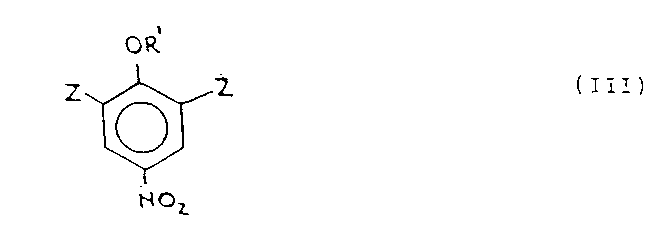 in the presence of a catalyst, to form a compound (IV) having the structure 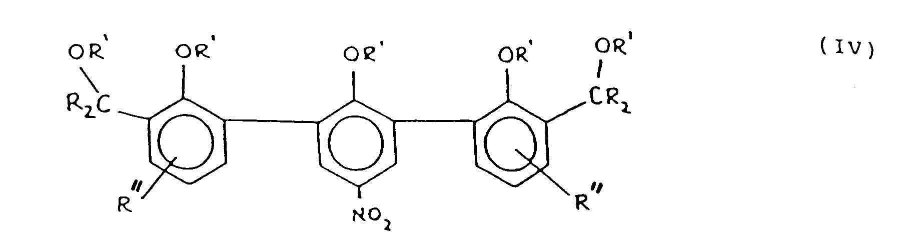
(b) subjecting the compound (IV) to reducing conditions to form a compound (V) having the structure 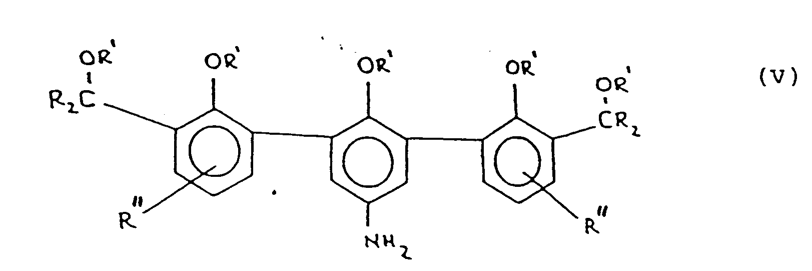
(c ) coupling the compound (V) to a compound having the structure 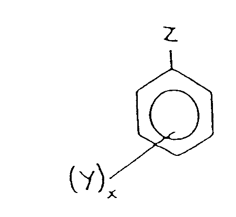 to form a compound (VI) having the structure 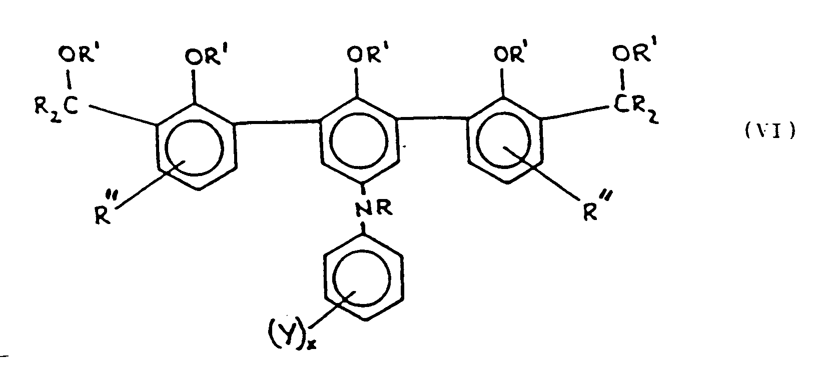
(d) halogenating the compound (VI) to form a compound (VII) having the structure 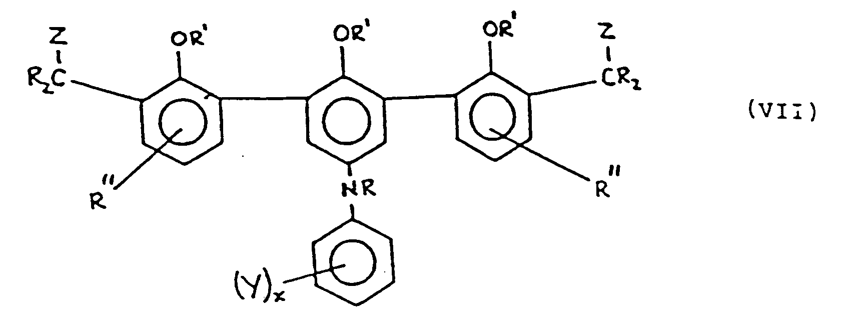
(e) forming the chromogenic cryptahemispherand (I) by coupling the compound (VII) with a diazacorand having the structure 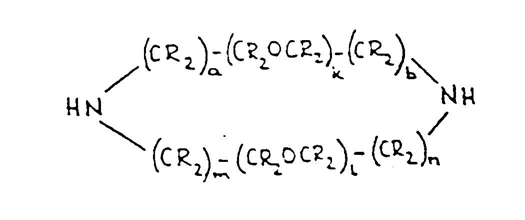 wherein each R is independently hydrogen, lower alkyl, lower alkylidene , lower alkenyl, allyl, aryl, or benzyl;
each R' is independently lower alkyl, lower alkylidene, lower alkenyl, allyl, aryl, or benzyl;
each R'' is independently hydrogen, lower alkyl, lower alkylidene, lower alkenyl, aryl or benzyl;
each Y is independently an electron withdrawing group;
each Z is halogen,
a is 1 to 3;
b is 1 to 3;
k is 1 to 3;
l is 1 to 3;
m is 1 to 3;
n is 1 to 3; and
x is 2 to 4.

2. A method according to claim 1, wherein compound (II) is prepared in a synthetic sequence comprising an alkylation or arylation reaction and a lithiation/boronation reaction in accordance with the reaction sequence:

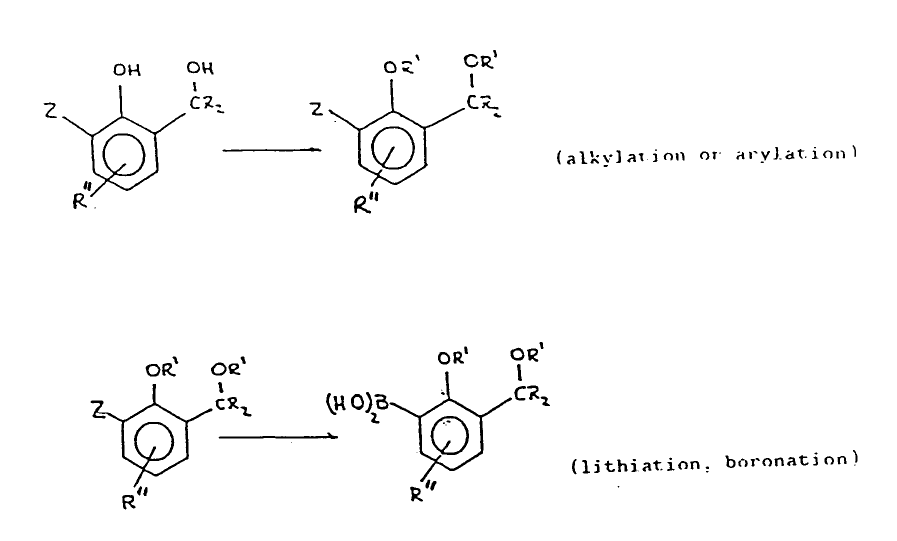

3. A method according to claim 1 or 2, wherein compound (III) is prepared by an alkylation or arylation reaction in accordance with the reaction sequence:

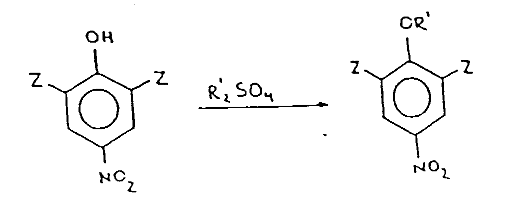

4. A method according to claim 1,2 or 3, in which in coupling step (a), the catalyst is tetrakis(triphenylphosphine)palladium.

## Patentansprüche

1. Verfahren zur Herstellung eines chromogenen Cryptahemispheranden (I) der Struktur 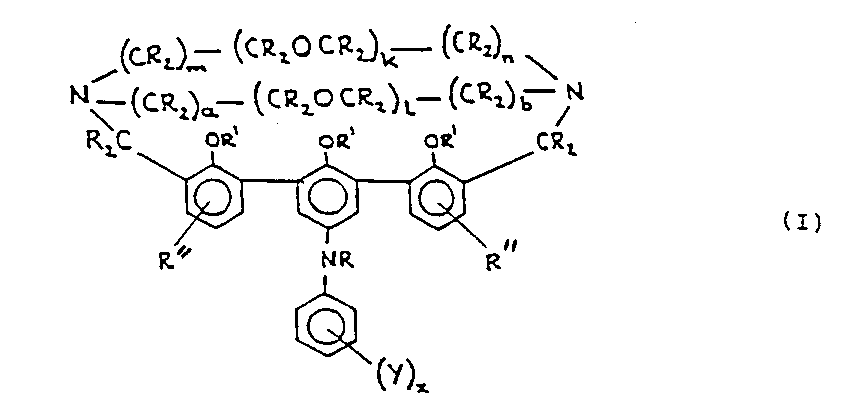 wobei das Verfahren die folgenden Stufen umfaßt:
(a) Kuppeln einer Verbindung (II) der Struktur 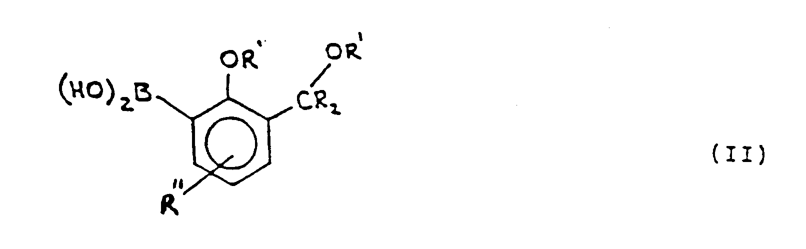 mit einer Verbindung (III) der Struktur 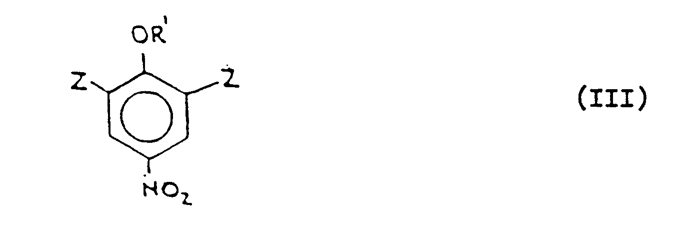 in Gegenwart eines Katalysators zu einer Verbindung (IV) der Struktur 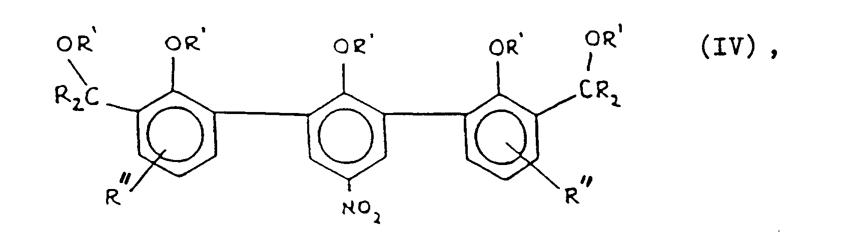
(b) Reduzieren der Verbindung (IV) zu einer Verbindung (V) der Struktur 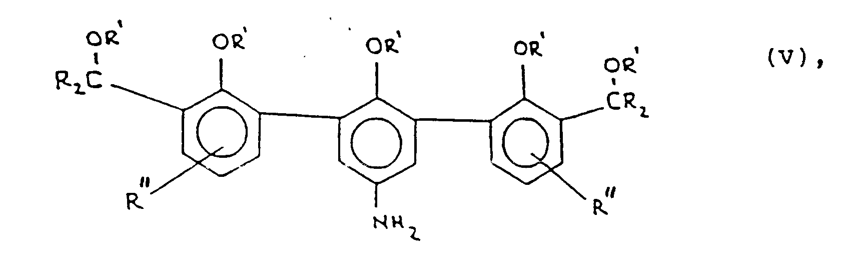
(c) Kuppeln der Verbindung (V) mit einer Verbindung der Struktur 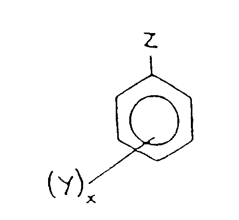 zu einer Verbindung (VI) der Struktur 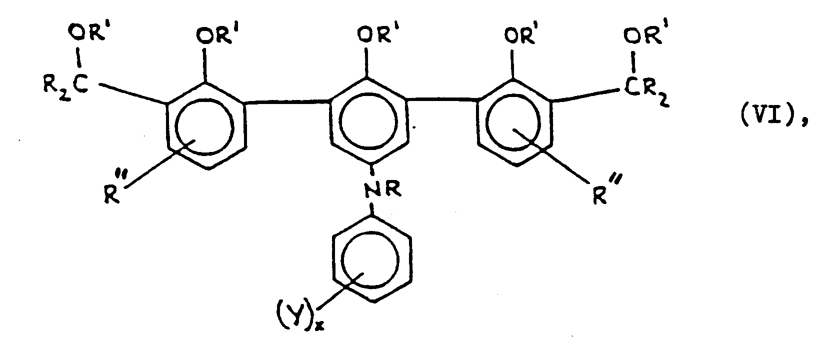
(d) Halogenieren der Verbindung (VI) zu einer Verbindung (VII) der Struktur 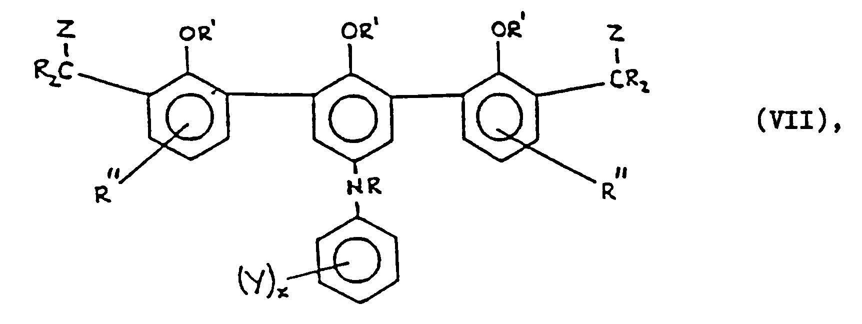
(e) Bilden des chromogenen Cryptahemispheranden (I) durch Kuppeln der Verbindung (VII) mit einem Diazacoranden der Struktur 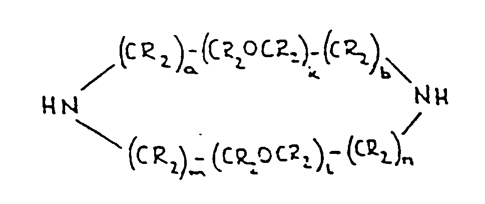 worin jeder der Reste R unabhängig voneinander ein Wasserstoffatom oder eine niedrigmolekulare Alkylgruppe, niedrigmolekulare Alkylidengruppe, niedrigmolekulare Alkenylgruppe, Allylgruppe, Arylgruppe oder Benzylgruppe;
jeder der Reste R' unabhängig voneinander eine niedrigmolekulare Alkylgruppe, niedrigmolekulare Alkylidengruppe, niedrigmolekulare Alkenylgruppe, Allylgruppe, Arylgruppe oder Benzylgruppe;
jeder der Reste R'' unabhängig voneinander ein Wasserstoffatom, eine niedrigmolekulare Alkylgruppe, niedrigmolekulare Alkylidengruppe, niedrigmolekulare Alkenylgruppe, Arylgruppe oder Benzylgruppe;
jeder der Reste Y unabhängig voneinander eine elektronenentziehende Gruppe;
jeder der Reste Z ein Halogenatom,
a 1 bis 3,
b 1 bis 3,
k 1 bis 3,
l 1 bis 3,
m 1 bis 3,
n 1 bis 3 und
x 2 bis 4 bedeuten.

2. Verfahren gemäß Anspruch 1, worin die Verbindung (II) in einer Reaktionsfolge hergestellt wird, die eine Alkylierung oder Arylierung und eine Lithiierung/Borierung gemäß den folgenden Gleichungen umfaßt:

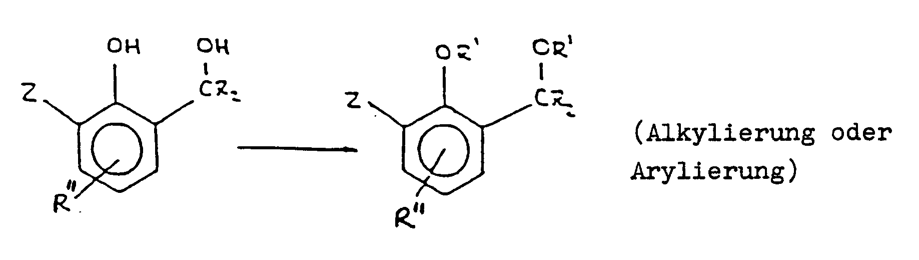

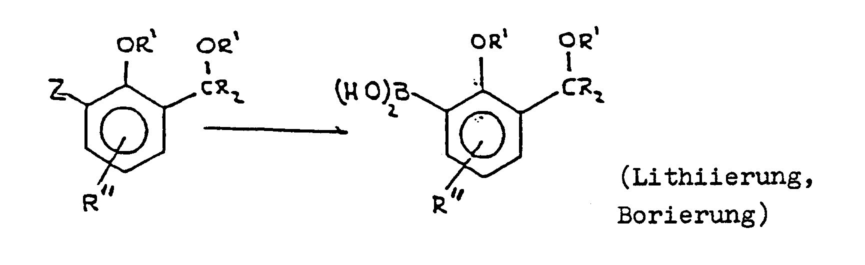

3. Verfahren gemäß Anspruch 1 oder 2, worin die Verbindung (III) durch eine Alkylierung oder Arylierung gemäß der folgenden Reaktionsgleichung hergestellt wird:

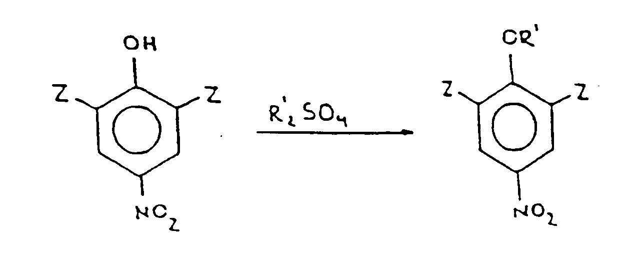

4. Verfahren gemäß Anspruch 1, 2 oder 3, worin in der Kupplungsstufe (a) der Katalysator Tetrakis-(triphenylphosphin)-palladium ist.

## Revendications

1. Procédé de préparation d'un cryptahémisphérand chromogénique (I) ayant la structure 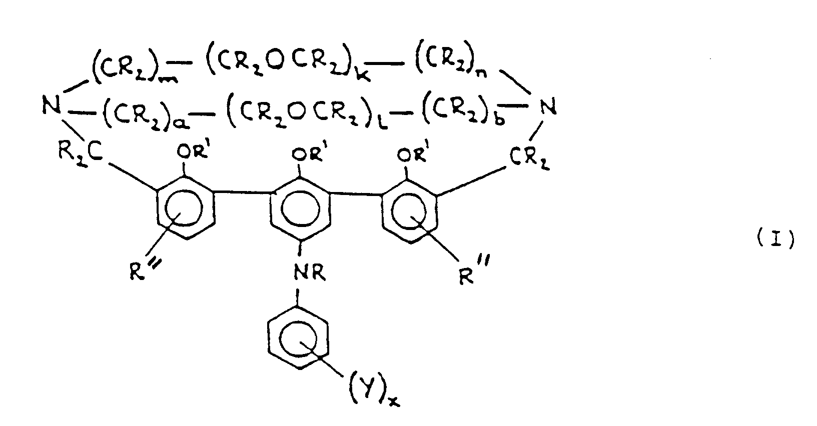 ledit procédé comprenant les étapes de :
(a) couplage d'un composé (II) ayant la structure 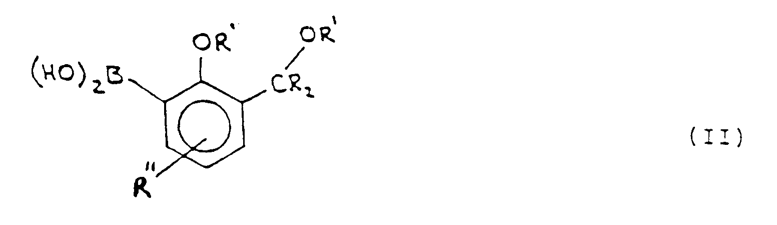 avec un composé (III) ayant la structure 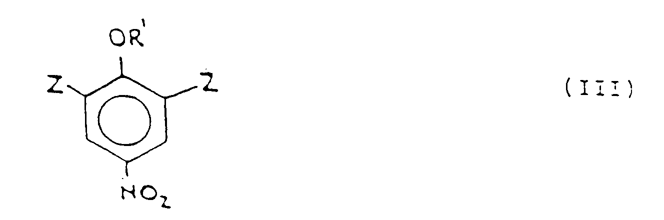 en présence d'un catalyseur, pour former un composé (IV) ayant la structure 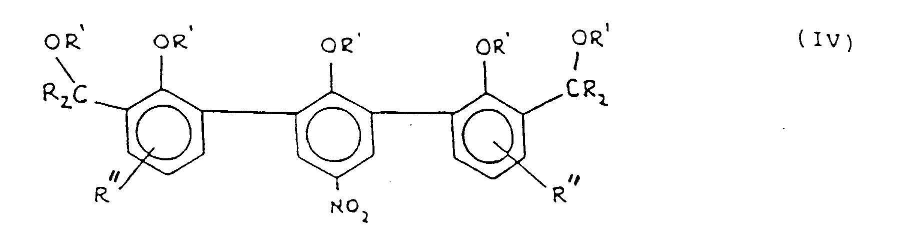
(b) soumission du composé (IV) à des conditions réductrices pour former un composé (V) ayant la structure 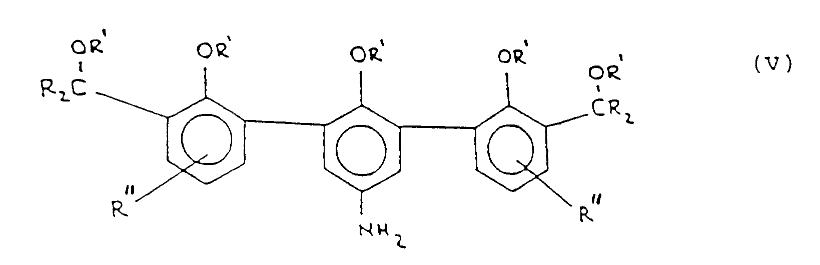
(c) couplage du composé (V) à un composé ayant la structure 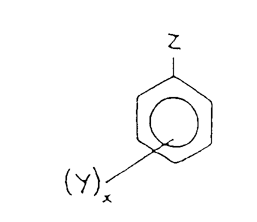 pour former un composé (VI) ayant la structure 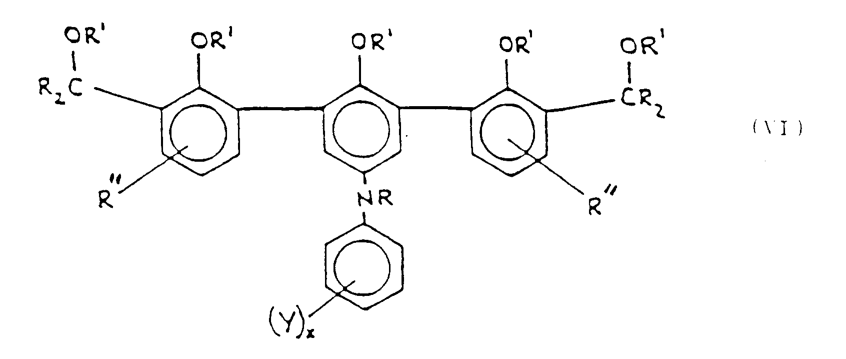
(d) halogénation du composé (VI) pour former un composé (VII) ayant la structure 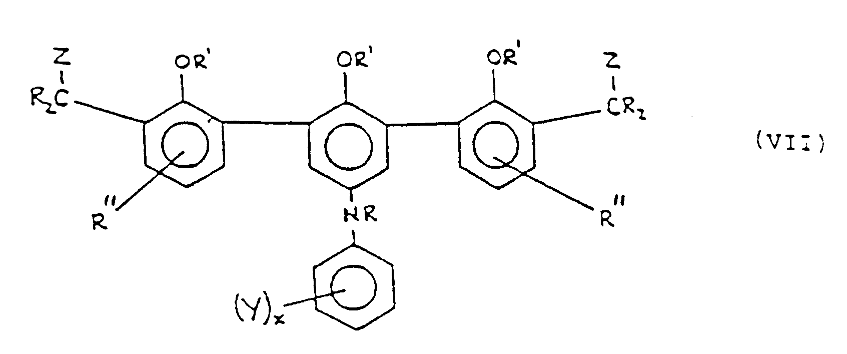
(e) formation du cryptahémisphérand chromogénique (I) par couplage du composé (VII) avec un diazocorand ayant la structure 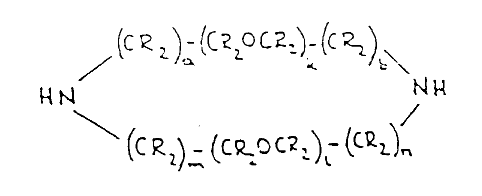 dans lequel chaque R est indépendamment un hydrogène, un alkyle inférieur, un alkylidène inférieur, un alkényle inférieur, un allyle, un aryle, ou un benzyle ;
chaque R' est indépendamment un alkyle inférieur, un alkylidène inférieur, un alkényle inférieur, un allyle, un aryle, ou un benzyle ;
chaque R'' est indépendamment un hydrogène, un alkyle inférieur, un alkylidène inférieur, un alkényle inférieur, un aryle, ou un benzyle ;
chaque Y est indépendamment un groupement supprimant les électrons ;
chaque Z est un halogène,
a est compris entre 1 et 3 ;
b est compris entre 1 et 3 ;
k est compris entre 1 et 3 ;
l est compris entre 1 et 3 ;
m est compris entre 1 et 3 ;
n est compris entre 1 et 3 ; et
x est compris entre 2 et 4.

2. Procédé selon la revendication 1, dans lequel le composé (II) est préparé selon une séquence synthétique comprenant une réaction d'alkylation ou d'arylation et une réaction de lithiation/boronation, selon la séquence de réactions :

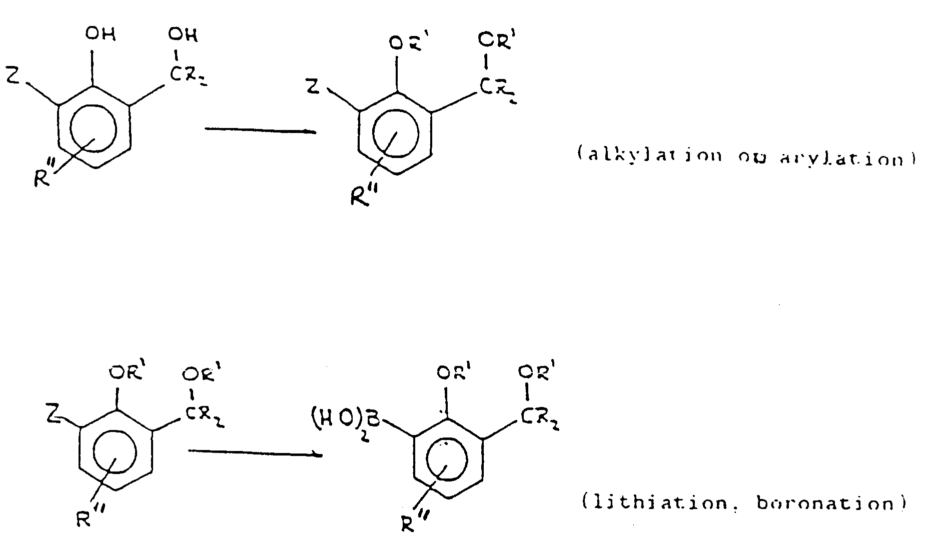

3. Procédé selon la revendication 1 ou la revendication 2, dans lequel le composé (III) est préparé par une réaction d'alkylation ou d'arylation selon la séquence de réactions :

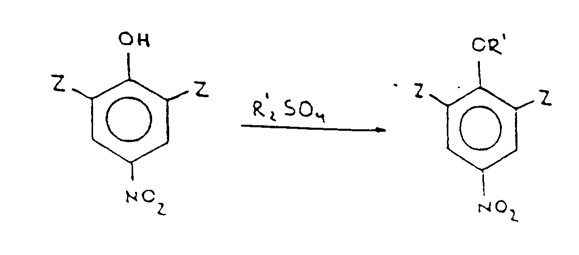

4. Procédé selon les revendications 1, 2 ou 3, dans lequel, dans l'étape de couplage, le catalyseur est le tétrakis(triphénylphosphine)palladium.
